# EUROPEAN PATENT SPECIFICATION

(11) **EP 3 997 239 B1**
(45) Date of publication and mention of the grant of the patent: **24.06.2026**
(21) Application number: 20839932.9
(22) Date of filing: 14.07.2020
(51) Int. Cl.: C12Q 1/04, C12Q 1/70, G01N 27/447, G01N 33/68, A61K 38/19, C12P 21/00

(54) **A PROCESS FOR SEPARATION AND QUANTITATION OF PROTEINS USING CAPILLARY ELECTROPHORESIS**
VERFAHREN ZUR TRENNUNG UND QUANTIFIZIERUNG VON PROTEINEN MITTELS KAPILLARELEKTROPHORESE
TRAITEMENT DE SÉPARATION ET DE QUANTIFICATION DE PROTÉINES PAR ÉLECTROPHORÈSE CAPILLAIRE

(30) Priority: 14.07.2019 IN 201921028239
(43) Date of publication of application: 18.05.2022
(73) Proprietor: Kashiv Biosciences, LLC, Bridgewater, NJ 08807 (US)
(72) Inventor: UPADHYAY, Roshan Ganeshlal, Ahmedabad 380061 (IN)
(74) Representative: Haseltine Lake Kempner LLP
(86) International application number: PCT/IB2020/056593
(87) International publication number: WO 2021/009669

(56) References cited:
- WO-A1-02/082066
- US-A1- 2017 007 712
- LACHER NATHAN A. ET AL: "Development, validation, and implementation of capillary gel electrophoresis as a replacement for SDS-PAGE for purity analysis of IgG2 mAbs : Electrodriven Separations", vol. 33, no. 2, 19 January 2010 (2010-01-19), DE, pages 218 - 227, XP093055093, ISSN: 1615-9306, Retrieved from the Internet <URL:https://api.wiley.com/onlinelibrary/tdm/v1/articles/10.1002%2Fjssc.200900597> DOI: 10.1002/jssc.200900597
- SALAS-SOLANO OSCAR ET AL: "Optimization and validation of a quantitative capillary electrophoresis sodium dodecyl sulfate method for quality control and stability monitoring of monoclonal antibodies", ANALYTICAL CHEMISTRY, AMERICAN CHEMICAL SOCIETY, US, vol. 78, no. 18, 15 September 2006 (2006-09-15), pages 6583 - 6594, XP002519390, ISSN: 0003-2700, [retrieved on 20060805], DOI: 10.1021/AC060828P
- OSCAR SALAS-SOLANO ET AL: "Optimization and Validation of a Quantitative Capillary Electrophoresis Sodium Dodecyl Sulfate Method for Quality Control and Stability Monitoring of Monoclonal Antibodies", ANALYTICAL CHEMISTRY, vol. 78, no. 18, 1 September 2006 (2006-09-01), US, pages 6583 - 6594, XP055551312, ISSN: 0003-2700, DOI: 10.1021/ac060828p
- TURNER, A ET AL.: "Development of orthogonal NISTmAb size heterogeneity control methods", ANALYTICAL AND BIOANALYTICAL CHEMISTRY, vol. 410, no. 8, March 2018 (2018-03-01), pages 2095 - 2110, XP036442990, DOI: 10.1007/s00216-017-0819-3
- SOBCZAK K., KRZYZOSIAK WLODZIMIERZ J: "RNA structure analysis assisted by capillary electrophoresis", NUCLEIC ACIDS RESEARCH, vol. 30, no. 22, 15 November 2002 (2002-11-15), pages 1 - 8, XP055803216, DOI: 10.1093/nar/gnf123

## Description

### Field of Disclosure

The present disclosure provides a method for analyzing, detecting and separating at least one low molecular weight impurity from a protein mixture using capillary electrophoresis, e.g., capillary electrophoresis - sodium dodecyl sulfate (CE-SDS). The present disclosure further provides methods to improve protein peak separation efficiency and quantification of a protein.

### Background

Capillary electrophoresis - sodium dodecyl sulfate (CE-SDS) is known in the art to quantify protein fragments. In a typical CE-SDS process, proteins bound with SDS migrate through a gel-filled capillary in an electric field. The samples are denatured and reduced with suitable reducing agents, e.g., DTT, TCEP, 2-mercaptoethanol. The SDS binds with the reduced proteins in a constant ratio such that the magnitude of charge on each protein fragment containing SDS (protein - SDS fragment) is directly proportional to its molecular weight. The resulting protein - SDS fragments are introduced into the gel-filled capillary and migrate from the cathode (negative) towards the anode (positive). Small molecules migrate through the gel-filled capillary more rapidly than large molecules therefore, the protein - SDS fragments are detected in order of increasing molecular weight.

Reduced CE-SDS method is widely known and used for characterization of antibody heterogeneity produced through product-related impurities like low molecular weight (truncated antibody) or high molecular weight (dimer antibody). However, characterization and detection of low molecular weight (LMW) impurities in a protein mixture wherein the protein of interest is pegylated which does not comprise an antibody or fragment thereof, using reduced CE-SDS, is challenging and has not been used.

The present disclosure provides methods for the characterization and detection of low molecular weight (LMW) impurities and high molecular weight (HMW), in a non-antibody protein mixture, with reduced CE-SDS and Non-reduced CE-SDS. The present disclosure employs a reduced CE-SDS method to improve the quality and purity of biopharmaceutical products. The present disclosure provides a robust process to access and evaluate product quality and purity in order to make these properties compatible with the requirements of regulatory agencies.

Product-related impurities are considered critical quality attributes in therapeutic protein products and need to be monitored across the drug development process, including in QC release testing.

### Summary of the Invention

The present invention provides an improved CE-SDS method of analyzing a protein mixture comprising protein of interest which is pegylated and does not contain an antibody (a non-antibody protein mixture) by separating LMW or HMW fragments present in the protein mixture.

Specifically, the present invention provides a process for improving the protein peak separation efficiency in CE-SDS, the process comprising:
a. Providing a protein mixture comprising a LMW impurity having a molecular weight of at least about 18 kDa, and a protein of interest having a molecular weight of about 38kDa;
b. Mixing the protein mixture with Tris, SDS and 2-mercaptoethanol to form a treated protein mixture;
c. Injecting the treated protein mixture in CE-SDS;
d. Performing the CE-SDS with capillary temperature is 15 °C to 20 °C to separate the LMW impurity from the protein having a molecular weight of about 38kDa; and
e. Separating the LMW impurity from the protein having a molecular weight of about 38kDa through CE-SDS.

In certain embodiments, the disclosure provides a method of performing CE-SDS at a separation voltage at about 15 kV or -15kV for at least about 35 minutes.

In certain embodiments, the CE-SDS is performed at an injection voltage of about 5 kV or -5kV for at least about 20 seconds.

### Brief Description of the Figures

Figure 1 depicts overlay electropherograms of samples tested at different capillary temperatures. The migration time reduced with increasing the temperature of capillary. The peak shape is improved at 15 °C and 20 °C compared to 25°C.
Figure 2 depicts Filgrastim (GCSF) peak elutes between 10 kDa and 20 kDa markers and PEG-Filgrastim (PEG-GCSF) main peak elutes between 35 kDa and 50 kDa molecular weight markers.
Figure 3 depicts reduced CE-SDS electropherograms of diluent, formulation buffer, 10 kDa molecular weight marker, PEG-GCSF without 10 kDa marker, PEG-GCSF with 10 kDa marker, Neulasta (RMP). GCSF (filgrastim) and GCSF spiked with PEG-GCSF to determine if there are any matrix interference and proteinaceous interference peaks eluting in the region of integration of main peak, and LMW species. No interference peaks were observed in the region of integration of the peaks of interest, main peak and LMW species.

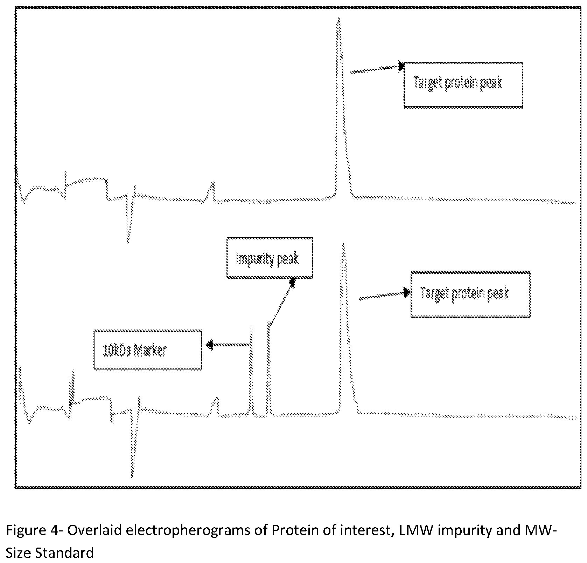

Figure 4 depicts overlaid electropherograms of PEG_Filgrastim DS treated protein mixture with and without 10 kDa marker (Zoomed).

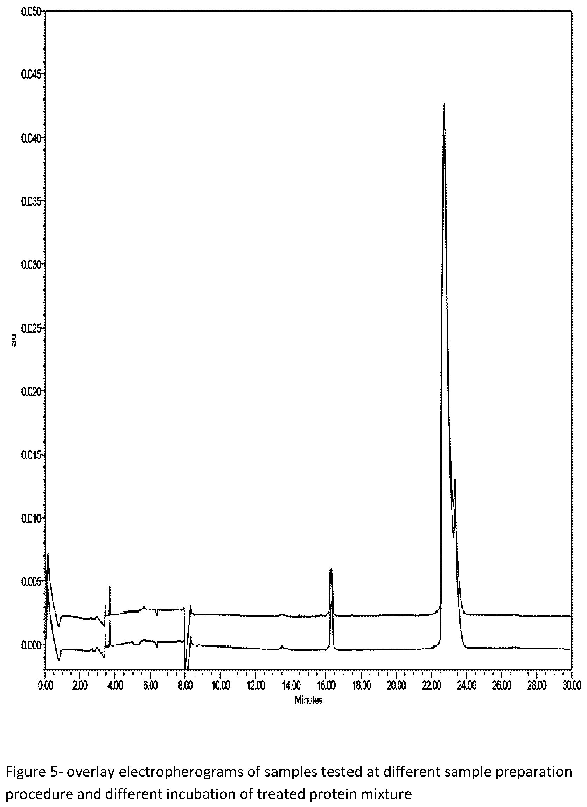

Figure 5 depicts overlay electropherograms of samples tested at different sample preparation procedure and different incubation of treated protein mixture.

### Detailed Description of the Invention

### Definitions

In order that the present disclosure can be more readily understood, certain terms are first defined. As used in this application, except as otherwise expressly provided herein, each of the following terms shall have the meaning set forth below. Additional definitions are set forth throughout the application.

As used herein, the term "protein of interest" is used in its broadest sense to include any protein (either natural or recombinant) with a desirable biological function. Such proteins of interest include, but are not limited to, enzymes, hormones, growth factors, cytokines and/or any fusion proteins PEGylated proteins, and any polymeric conjugate of the protein. In embodiment the protein of interest is pegylated GCSF having molecular weight about 38kDa or 39 kDa.

The term about 38 kDa is interchangeable with 38 kDa or 39 kDa. The difference in the molecular weight is due to pegylation.

In certain embodiments, the protein of interest has a molecular weight of between about 10 kDa and about 150 kDa. In certain embodiments, the protein of interest has a molecular weight of between about e 25 kDa and about 150 kDa. In certain embodiments, the protein of interest has a molecular weight of at least about 38 kDa. In certain embodiments, the protein of interest is present in a mixture, for which purification is desired.

The terms "protein mixture" and "sample," as used interchangeably herein, include a protein mixture comprises protein of interest and suitable buffer or water/ aqueous solution and does not contain any antibody. In certain embodiment the protein mixture does not contain antibody and fragment thereof. In certain embodiments, the protein mixture comprises a plurality of proteins and protein of interest. In certain embodiments, the protein mixture optionally comprises low molecular weight impurity.

In certain embodiments, the protein mixture comprises of a protein having a molecular weight of between about 5 kDa and about 150 kDa. In certain embodiments, the impurity is a LMW impurity, a HMW impurity, an oxidized protein, or a deamidated protein.

The term "HMW species" refers to any one or more proteins with a higher apparent molecular weight relative to the intact protein of interest. The HMW species can be unrelated to the protein of interest or are aggregates, e.g., a dimer or multimer or any combination of the intact protein and any fragment thereof. In certain embodiments, the two proteins or protein fragments with different molecular weights may migrate together during electrophoresis because of non-uniform charge density due to non-uniform detergent coating of the proteins. In certain embodiment the HMW has molecular weight higher than about 38kDa.

The term "LMW species" refers to one or more proteins with a lower apparent molecular weight relative to the intact protein of interest. The LMW species can be unrelated to the protein of interest or can be protein fragments. In certain embodiment the LMW has molecular weight lower than about 38kDa. In one aspect of such embodiment the LMW is selected from 5 kDa, 6kDa, 7 kDa, 8kDa, 9 kDa, 10 kDa, 11 kDa, 12 kDa, 13 kDa, 14kDa, 15kDa, 16kDa, 17kDa,18 kDa, 19kDa, 20 kDa, 21kDa, 22kDa, 23kDa,24 kDa, 25kDa, 26kDa, 27kDa, 28kDa, 29kDa to 30 kDa. In certain embodiment the LMW is unpegylated GCSF. Unpegylated GCSF molecular weight is about 18kDa. In certain embodiment the LMW is derived from pegylated GCSF or LMW is a fragment of Pegylated GCSF. In certain embodiment the LMW is present in protein mixture obtained from at least any one of previous steps of downstream, upstream, and stability. In certain embodiment the LMW is spiked in the protein mixture to verify the accuracy of detection of CE-SDS method. In certain embodiment the LMW spiked protein mixture and non-spiked LMW protein mixture are tested and compared to analyze the presence of LMW in non-spiked protein mixture.

The terms "treated protein mixture" include a protein mixture and detergent which is capable to denature the protein in mixture. In certain embodiment the detergent is sodium dodecyl sulphate (SDS). Optionally protein mixture is further treated with reducing agent capable of reducing the non-covalent bonds of proteins. Reducing agent is selected from DTT, B-mercaptoethanol and TCEP. Optionally protein mixture is further mixed with suitable marker having molecular weight selected from 10 kDa to 250kDa. In certain embodiment marker's molecular weight helps in quantifying or detecting the LMW or HMW in protein mixture.

In certain embodiment the treated protein mixture comprises protein mixture mixed with detergent, reducing agent and optionally suitable marker.

The terms "separating" or "isolating," as used interchangeably herein, refer to increasing the degree of purity of a protein of interest from a composition or sample comprising the protein of interest and one or more impurities. The separation or isolation can occur via electrophoresis, wherein an electric field is used to move negatively charged particles through a substance. When the particles have the same uniform electric charge, they migrate through the electric field based on their size, and the differential migration rates cause the particles, including proteins of interest, to be separated or isolated.

Capillary gel electrophoresis (CGE) using sodium dodecyl sulfate, commonly referred as CE-SDS, is the capillary format of polyacrylamide gel electrophoresis (SDS- PAGE), wherein the polyacrylamide slab gel is replaced with a narrow-bore glass capillary filled with a replaceable polymer sieving matrix. In contrast to traditional SDS-PAGE, CE-SDS can measure the overall fragmentation pattern and perform accurate protein quantitation with advantages including direct on-column UV or fluorescence detection, automation, and enhanced resolution and reproducibility.

SDS has been extensively utilized as the default detergent in both SDS-PAGE and CE-SDS techniques for protein denaturation, which can be attributed to a widely accepted consensus that protein tends to bind a relatively constant amount of SDS on a weight basis resulting in uniform mass/charge ratios of SDS-protein complexes in most cases. Consequently, intrinsic polypeptide charges of protein molecules become negligible and the final separation depends entirely on differences in the relative molecular mass of their denatured polypeptides.

The terms "detergent", "surfactant" and "foaming agent" are used interchangeably herein. A detergent is a surfactant or a mixture of surfactants; when present in small amounts, a surfactant reduces the surface tension of a liquid (reduces the work needed to create the foam) or increases its colloidal stability by inhibiting coalescence of bubbles. A detergent reduces non-covalent interactions in proteins.

The term "quantification and/or detection" refers to proper peak separation obtained through CE-SDS for evaluation of fragments or size variants which are HMW or LMW compared to protein of interest. Proper peak separation does not have interference peak or peak broadening. Furthermore, peak separation is evaluated by parameters of Specificity, Linearity, Accuracy and Precision. In certain embodiment the quantification and/or detection is obtained through reduced CE-SDS.

The present invention generally provides a method of analyzing a protein mixture, which does not contain any antibody or any fragment thereof, by separating components of the protein mixture in reduced CE-SDS.

The method of the present invention is defined in claim 1.

In one aspect, the present invention performs CE-SDS at capillary temperature below 20°C. In certain embodiments the capillary temperature is selected from 15°C, 16°C, 17°C, 18°C, 19°C and 20°C. In the method of the invention, the quantification and/or detection of low molecular weight or high molecular weight impurity is improved compared to CE-SDS performed at 25°C.

In an embodiment the suitable buffer used in treated protein mixture is Tris buffer. In preferred embodiment the capillary temperature is about 15°C to about 17°C. In an embodiment the preferred capillary temperature is about 15°C.

In Pegylated proteins, PEG is attached to the protein and may interfere in elution with main peak. To reduce the impact of PEGylation, viscosity of separation conditions inside the capillary changed by changing the cartridge temperature. The effect of temperature on separation was evaluated and improved separation of peak is obtained at temperature at 20°C and 15°C compared to reduced CE-SDS performed at 25°C.

In certain embodiments, the disclosure provides a method of performing CE-SDS at a separation voltage at about 15 kV for at least about 35 minutes.

In certain embodiments, the CE-SDS is performed at an injection voltage of about 5 kV for at least about 20 seconds. In certain embodiments, the CE-SDS is performed with Tris buffer or HEPES buffer.

In certain embodiments, the CE-SDS is performed at a pH of between about 6 and about 10. In certain embodiments, the CE-SDS is performed with Tris buffer at pH 9.

In certain embodiments, the CE-SDS is performed at separation voltage at about 15 kV, for at least about 30 minutes. In certain embodiments, the CE-SDS is performed at a separation voltage of about 15 kV, for at least about 35 minutes.

The migration time of eluting peaks depends on separation voltage which also helps in resolving the peaks. The presence invention is not bound to use of separation voltage 15 kV as the optimizing the separation voltage and its respective effect on the elution of the peaks are well known to the skilled person.

In certain embodiments, the CE-SDS is performed at an injection voltage of about 5 kV for at least about 10 seconds. In certain embodiments, the CE-SDS is performed at an injection voltage of about -5 kV, between about 0 and about 15 seconds.

In certain embodiments, the treated protein mixture is injected at a room temperature of about 25°C.

According to the invention, the capillary temperature is between about 15°C and about 20°C. In certain embodiments, the capillary temperature is between about 15°C and about 18°C. In certain embodiments, the capillary temperature is about 15°C.

In certain embodiments, the protein mixture does not contain any antibody or a fragment thereof. In certain embodiments, the protein mixture does not contain a full-length antibody having a molecular weight of about 150 kDa but can contain an antibody fragment having a molecular weight of less than about 100 kDa.

In certain embodiments, the protein mixture comprises a fusion protein comprising an antibody fragment. In certain embodiments, the protein mixture comprises a fusion protein comprising an antibody fragment attached with PEG.

### Preparation of protein mixture and Treated protein mixture

The protein mixture comprises protein of interest and suitable buffer or water/ aqueous solution and does not contain any antibody. In certain embodiment the protein mixture does not contain antibody and fragment thereof. In certain embodiments, the protein mixture comprises a plurality of proteins and protein of interest. The plurality of proteins is selected from fragments and protein except protein of interest. It may form LMW or HMW impurity. In certain embodiments, the protein mixture optionally comprises low molecular weight impurity.

In certain embodiment the LMW is present in protein mixture obtained from at least any one of previous steps of downstream, upstream, and stability. In certain embodiment the LMW is spiked in the protein mixture to verify the accuracy of detection of CE-SDS method. In certain embodiment the LMW spiked protein mixture and non-spiked LMW protein mixture are tested and compared to analyze the presence of LMW in non-spiked protein mixture.

In an embodiment the protein mixture is at least about 2mg/ml. In certain embodiment the protein mixture is selected from about 2mg/ml, about 3 mg/ml, about 4mg/ml, about 5mg/ml. In an embodiment the protein mixture is at least about 5mg/ml.

In certain embodiments, the treated protein mixture is prepared by mixing a protein mixture in a suitable buffer comprising a detergent, and a suitable reducing agent. In certain embodiments, the reducing agent is DTT, B-mercaptoethanol, or TCEP. In certain embodiments, the selection of reducing agent depends on the protein of interest present in the protein mixture.

The suitable buffer is Tris buffer at pH 9. Tris concentration is selected from 60mM to 90mM.

The present invention provides examples for illustration purpose only, and the scope of the invention should not be considered limited by the presented examples.

### Example 1

Pegfilgrastim is generated by covalently attaching a 20 kDa polyethylene glycol (PEG) moiety to the amino terminus of filgrastim produced in *Escherichia coli.* Pegfilgrastim keeps the same biological activity as filgrastim but has a longer circulating half-life. Pegfilgrastim has a total molecular weight of 39,000 Daltons and is indicated for reducing the incidence of infection.

For the reduced CE-SDS method for Peg-filgrastim, the Specificity was assessed from injection of diluent (water), formulation buffer, and oxidized Peg-filgrastim sample. Electropherograms of diluent and formulation buffer are evaluated to determine if there are any proteinaceous interference peaks eluting in the region of integration of main peak, and LMW species. No interference peaks are observed in the region of integration of the peaks of interest, main peak, and the LMW species.

### Evaluation of cartridge temperature and sample preparation

The initial separation conditions for CE-SDS consist of separation voltage (-15 kV for 35 or 40 minutes), injection voltage (-5 kV for 20 seconds), PDA detector wavelength (220 nm), sample temperature (25°C), and capillary temperature (25°C). Since the PEG attached in Pegylated protein might be interfering in the elution of main peak. To reduce the impact of PEGylation, viscosity of separation conditions inside the capillary changed by changing the cartridge temperature. The effect of temperature on separation is evaluated by changing the capillary temperature from 25°C to 20°C and 15°C.

Use of master mix was also removed by adding each component (sample buffer, 10 kDa marker and 2-mercaptoethanol) individually in the sample preparation. As it is known that Peg-filgrastim (~12.5 mg/mL) and its syringe(~10 mg/mL) have a much higher concentration than 0.50 mg/mL, the sample is prepared as 5 mg/mL in DI water initially and then 10 µL of 5 mg/mL sample added into 83 µL of sample buffer, mixed it well. After that 2 µL of 10 kDa marker and 5 µL of 2-mercptoethanol are added into the solution. The sample is centrifuged at 14 k rpm for 10 minutes at 15 °C of temperature and then incubated at 55 °C for 5 minutes. The sample is then cooled down at room temperature for 5 minutes and then finally centrifuged at 14 k rpm for 10 minutes at 15 °C of temperature before the analysis.

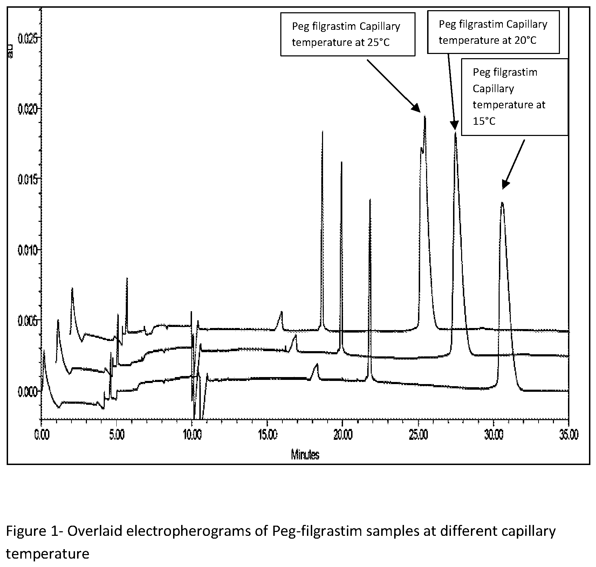

Figure 1 shows the overlay electropherograms of samples tested at different capillary temperatures. The migration time reduced with increasing the temperature of capillary. The peak shape is improved as the temperature is decreased from 25 °C to 15 °C.

Based on the Figure 1, 15°C temperature is more suitable for the method.

### Evaluation of molecular weight

To check the migration time for Peg-filgrastim and filgrastim peaks with respect to their molecular weights, protein molecular weight size standard was injected along with filgrastim and Peg-filgrastim processed samples.

The protein size standard contains seven proteins of 10, 20, 35, 50, 100, 150, and 225 kDa. The Peg-filgrastim (~39 kDa) peak should elute between peak 3 and peak 4 having molecular weights of 35 and 50 respectively.

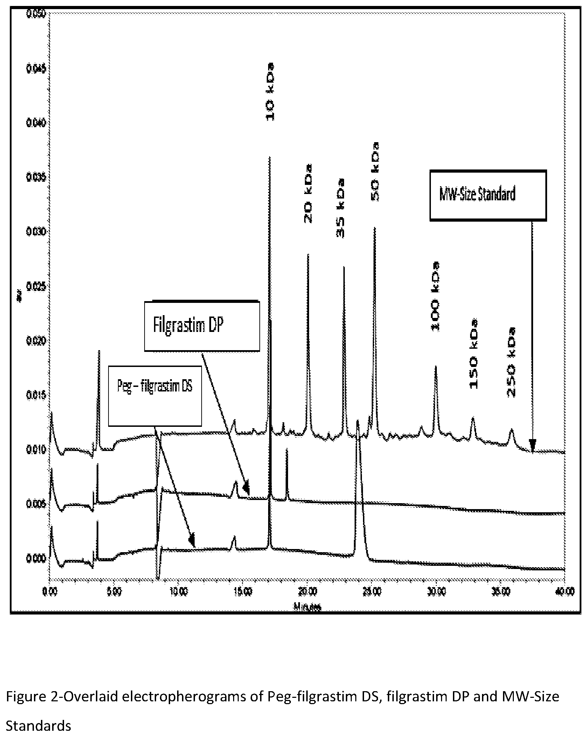

As Figure 2 shows, the filgrastim peak is eluting between 10 kDa and 20 kDa markers and Peg-filgrastim main peak is eluting between 35 kDa and 50 kDa molecular weight markers. It confirms that the filgrastim peak and Peg-filgrastim main peak are eluting with respect to their molecular size and shape.

The use of a 10 kDa molecular weight marker is employed as an internal control, mainly to check the starting window of integration since filgrastim elutes after the marker peak and also to check the method capability to resolve species having minor difference in their molecular weights. In this case marker peak having 10 kDa molecular weight and filgrastim peak which is having ~ 18kDa molecular weight.

Since the concentration of Peg-filgrastim DP is ~ 10 mg/mL and DS is more than 10 mg/mL, the sample preparation step starts with making of 5 mg/mL of stock sample using DI water or equivalent. After that sample is prepared by adding 10 µL of 5mg/mL sample into 83 µL of sample buffer, 2 µL of 10 kDa marker, and 5 µL of 2-mercaptoethanol to make the final volume of 100 µL (The component of sample preparation may change proportionally, according to the final volume).

The sample is centrifuged at 14 k rpm for 10 minutes at 15 °C of temperature and then incubate at 55 °C for 5 minutes. The sample is cooled down at room temperature for 5 minutes and then finally centrifuge at 14 k rpm for 10 minutes at 15 °C of temperature before the analysis.

Based on the selected conditions, the method performance was evaluated. The following parameters were investigated, Specificity, linearity, accuracy, precision, and the LOQ & LOD determined from a spiked sample.

**Table 1: Time Program in Separation Method**

| **Time (min)** | **Event** | **Value** | **Duration** | **Inlet vial** | **Inlet tray** | **Outlet vial** | **Outlet tray** |
|---|---|---|---|---|---|---|---|
| | Rinse pressure | 4854.1 | 3.00 min | D1 | Buffer | D1 | Buffer |
| | Rinse pressure | 4854.1 | 1.00 min | E1 | Buffer | E1 | Buffer |
| | Rinse pressure | 4854.1 | 1.00 min | F1 | Buffer | F1 | Buffer |
| | Rinse pressure | 4854.1 | 10.00 min | B1 | Buffer | B1 | Buffer |
| | Wait | | 0.00 min | A1 | Buffer | A1 | Buffer |
| | Wait | | 0.00 min | A4 | Buffer | A4 | Buffer |
| | Inject Voltage | -5.0 kV | 15.0 S | A0 | Buffer | C1 | Buffer |
| | Wait | | 0.00 min | B4 | Buffer | B4 | Buffer |
| 0.00 | Separation Voltage | -15.0 kV | 35.00 min | C1 | Buffer | C1 | Buffer |
| 5.00 | Autozero | | | | | | |
| 35.00 | End | | | | | | |

### Example 2

Specificity refers to the ability to assess unequivocally the analyte in the presence of components which may be expected to be present. Specificity of method should be assessed by checking matrix interference and by evaluating separation selectivity of the method.

For reduced CE-SDS method for PEG-Filgrastim, the Specificity was assessed from:
1. DI water (Blank) - To check the matrix interference
2. Formulation buffer of Peg-filgrastim - To check the matrix interference
3. 10 kDa molecular weight marker - To check the matrix interference
4. Peg-filgrastim DS without 10 kDa marker - To check the separation selectivity
5. Peg-filgrastim DS with 10 kDa marker (control) - To check the separation selectivity
6. Neulasta (RMP) - To check the separation selectivity
7. filgrastim - To check the separation selectivity
8. DS spiked with filgrastim - To check the separation selectivity

Electropherograms of diluent and formulation buffer were evaluated to determine if there are any proteinaceous interference peaks eluting in the region of integration of main peak, and LMW species. No interference peaks were observed in the region of integration of the peaks of interest, main peak and LMW species.

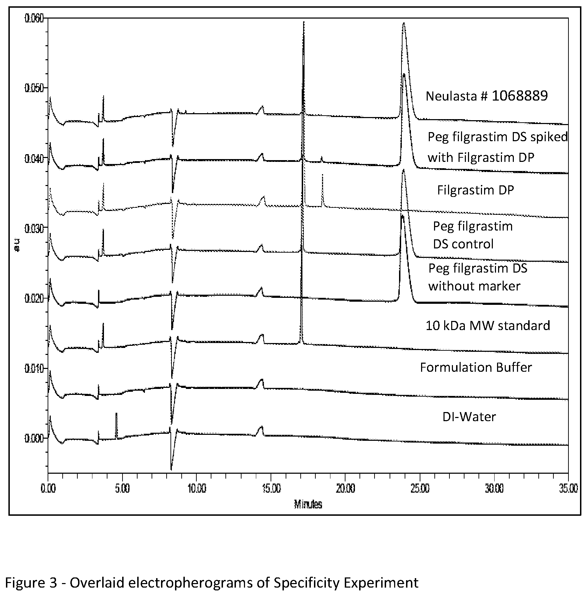

Figures 3 show Electropherograms of all the aforementioned components. The 10 kDa marker peak is very well separated from the peak of GCSF and the GCSF peak is well separated from the Peg-filgrastim peak. This proves that the method has separation selectivity and the resolution of the method is also at par to resolve the peaks.

### Example 3

### Non-reduced CE-SDS method description for PEG-GCSF

The sample preparation step starts with making of 1.5 mg/mL of stock sample using DI water or equivalent. After that sample is prepared by adding 15 µL of 1.5mg/mL sample into 95 µL of master mix, which contains 64 µL of sample buffer, 29.5 µL of DI water and 1.5 µL of 10 kDa marker. The sample is centrifuged at 14 k rpm for 10 minutes at 15 °C of temperature and then incubate at 55 °C for 5 minutes. The sample is cooled down at room temperature for 5 minutes and then finally centrifuge at 14.8 k rpm for 5 minutes at ambient temperature before the analysis.

Capillary temperature: 25°C

**Table 2: Time Program in Separation Method**

| **Time (min)** | **Event** | **Value** | **Duration** | **Inlet vial** | **Inlet tray** | **Outlet vial** | **Outlet tray** |
|---|---|---|---|---|---|---|---|
| | Rinse pressure | 4854.1 | 3.00 min | D1 | Buffer | D1 | Buffer |
| | Rinse pressure | 4854.1 | 1.00 min | E1 | Buffer | E1 | Buffer |
| | Rinse pressure | 4854.1 | 1.00 min | F1 | Buffer | F1 | Buffer |
| | Rinse pressure | 4854.1 | 10.00 min | B1 | Buffer | B1 | Buffer |
| | Wait | | 0.00 min | A1 | Buffer | A1 | Buffer |
| | Wait | | 0.00 min | A4 | Buffer | A4 | Buffer |
| | Inject Voltage | -5.0 kV | 20.0 S | A0 | Buffer | C1 | Buffer |
| | Wait | | 0.00 min | B4 | Buffer | B4 | Buffer |
| 0.00 | Separation Voltage | -15.0 kV | 30.00 min | C1 | Buffer | C1 | Buffer |
| 5.00 | Autozero | | | | | | |
| 30.00 | End | | | | | | |

## Claims

1. A process for an improving protein peak separation efficiency in CE-SDS comprising:
a. Providing a protein mixture comprising a LMW impurity having a molecular weight of at least about 18 kDa, and a protein of interest having a molecular weight of about 38kDa;
b. Mixing the protein mixture with Tris, SDS and 2-mercaptoethanol to form a treated protein mixture;
c. Injecting the treated protein mixture in CE-SDS;
d. Performing the CE-SDS with capillary temperature is 15 °C to 20 °C to separate the LMW impurity from the protein having a molecular weight of about 38kDa; and
e. Separating the LMW impurity from the protein having a molecular weight of about 38kDa through CE-SDS.

2. The process according to claim 1 wherein the SDS concentration is 1%.

3. The process according to claim 1 wherein the treated protein mixture injected into CE-SDS capillary is about 0.5 mg/ml.

4. The process according to claim 1 wherein the suitable temperature of CE-SDS capillary is 15 °C to 20 °C, optionally selected from 15°C, 16°C, 17°C, 18°C, 19°C, or 20°C.

5. The process according to claim 1 wherein the CE-SDS is performed at separation voltage at about 15 kV or -15kV for at least about 30 minutes and/or at an injection voltage of about 5 kV or -5kV for at least about 10 seconds.

6. The process according to claim 1 wherein the CE-SDS is performed with Tris buffer at pH 9, optionally at a Tris concentration is selected from 60mM to 90mM.

7. The process according to claim 1 wherein the low molecular weight impurity is GCSF or any other fragments derived from PEG-GCSF.

8. The process according to claim 1 wherein the protein mixture comprises an antibody fragment having a molecular weight of less than about 100 kDa.

## Patentansprüche

1. Verfahren zum Verbessern einer Proteinpeaktrenneffizienz in CE-SDS, umfassend:
a. Bereitstellen eines Proteingemischs, umfassend eine LMW-Verunreinigung, die ein Molekulargewicht von mindestens etwa 18 kDa aufweist, und ein Zielprotein, das ein Molekulargewicht von etwa 38 kDa aufweist;
b. Mischen des Proteingemischs mit Tris, SDS und 2-Mercaptoethanol, um ein behandeltes Proteingemisch zu bilden;
c. Injizieren des behandelten Proteingemischs in CE-SDS;
d. Durchführen der CE-SDS mit einer Kapillartemperatur von 15 °C bis 20 °C, um die LMW-Verunreinigung von dem Protein, das ein Molekulargewicht von etwa 38 kDa aufweist, zu trennen; und
e. Trennen der LMW-Verunreinigung von dem Protein, das ein Molekulargewicht von etwa 38 kDa aufweist, durch CE-SDS.

2. Verfahren nach Anspruch 1, wobei die SDS-Konzentration 1 % beträgt.

3. Verfahren nach Anspruch 1, wobei das in die CE-SDS-Kapillare injizierte behandelte Proteingemisch etwa 0,5 mg/ml beträgt.

4. Verfahren nach Anspruch 1, wobei die geeignete Temperatur der CE-SDS-Kapillare 15 °C bis 20 °C beträgt, optional ausgewählt aus 15 °C, 16 °C, 17 °C, 18 °C, 19 °C oder 20 °C.

5. Verfahren nach Anspruch 1, wobei die CE-SDS bei einer Trennspannung von etwa 15 kV oder -15 kV für mindestens etwa 30 Minuten und/oder bei einer Injektionsspannung von etwa 5 kV oder -5 kV für mindestens etwa 10 Sekunden durchgeführt wird.

6. Verfahren nach Anspruch 1, wobei die CE-SDS mit Tris-Puffer bei pH 9 durchgeführt wird, optional bei einer Tris-Konzentration, die aus 60 mM bis 90 mM ausgewählt ist.

7. Verfahren nach Anspruch 1, wobei die niedermolekulare Verunreinigung GCSF oder beliebige andere von PEG-GCSF abgeleitete Fragmente ist.

8. Verfahren nach Anspruch 1, wobei das Proteingemisch ein Antikörperfragment umfasst, das ein Molekulargewicht von weniger als etwa 100 kDa aufweist.

## Revendications

1. Traitement d'amélioration de l'efficacité de séparation des pics de protéines en CE-SDS comprenant :
a. la fourniture d'un mélange de protéines comprenant une impureté LMW de faible poids moléculaire ayant un poids moléculaire d'au moins environ 18 kDa, et une protéine d'intérêt ayant un poids moléculaire d'environ 38 kDa ;
b. le mélange du mélange de protéines avec du Tris, du SDS et du 2-mercaptoéthanol pour former un mélange de protéines traité ;
c. l'injection du mélange de protéines traité dans du CE-SDS ;
d. la réalisation du CE-SDS à une température capillaire comprise entre 15 °C et 20 °C afin de séparer l'impureté LMW de faible poids moléculaire de la protéine ayant un poids moléculaire d'environ 38 kDa; et
e. la séparation de l'impureté de faible poids moléculaire de la protéine ayant un poids moléculaire d'environ 38 kDa par CE-SDS.

2. Traitement selon la revendication 1, dans lequel la concentration de SDS est de 1 %.

3. Traitement selon la revendication 1 dans lequel le mélange de protéines traité injecté dans le capillaire CE-SDS est d'environ 0,5 mg/ml.

4. Traitement selon la revendication 1 dans lequel la température appropriée du capillaire CE-SDS est de 15 °C à 20 °C, éventuellement choisie parmi 15 °C, 16 °C, 17 °C, 18 °C, 19 °C ou 20 °C.

5. Traitement selon la revendication 1, dans lequel le CE-SDS est réalisé à une tension de séparation d'environ 15 kV ou - 15 kV pendant au moins environ 30 minutes et/ou à une tension d'injection d'environ 5 kV ou -5 kV pendant au moins environ 10 secondes.

6. Traitement selon la revendication 1 dans lequel le CE-SDS est réalisé avec un tampon Tris à pH 9, éventuellement à une concentration de Tris sélectionnée entre 60 mM et 90 mM.

7. Traitement selon la revendication 1, dans lequel l'impureté de faible poids moléculaire est du GCSF ou tout autre fragment dérivé du PEG-GCSF.

8. Traitement selon la revendication 1, dans lequel le mélange de protéines comprend un fragment d'anticorps ayant un poids moléculaire inférieur à environ 100 kDa.
